(19) Europäisches Patentamt
European Patent Office
Office européen des brevets

(11) Veröffentlichungsnummer: 0 259 268
A1

# (12) EUROPÄISCHE PATENTANMELDUNG

(21) Anmeldenummer: 87810497.5

(22) Anmeldetag: 31.08.87

(51) Int. Cl.4: C 07 F 7/18
C 07 D 205/08, C 07 D 499/00

(30) Priorität: 04.09.86 CH 3562/86

(43) Veröffentlichungstag der Anmeldung:
09.03.88 Patentblatt 88/10

(84) Benannte Vertragsstaaten:
BE CH DE ES FR GB IT LI NL SE

(71) Anmelder: CIBA-GEIGY AG
Klybeckstrasse 141
CH-4002 Basel (CH)

(72) Erfinder: Hungerbühler, Ernst, Dr.
Zeisigweg 4
CH-4310 Rheinfelden (CH)

(54) **Verfahren zur Herstellung optisch aktiver Beta-Lactame.**

(57) Die Erfindung betrifft ein Verfahren zur Herstellung von β-Lactamverbindungen der Formel

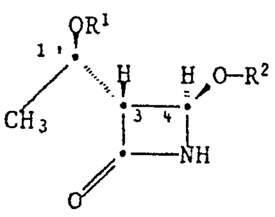

(I),

worin $R^1$ Wasserstoff oder eine unter den Ringschlussbedingungen nicht abspaltbare Hydroxyschutzgruppe $R^{1a}$ und $R^2$ den Acylrest einer schwachen Carbonsäure darstellt, dadurch gekennzeichnet, dass man eine Verbindung der Formel

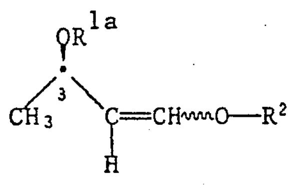

(II),

worin $R^{1a}$ und $R^2$ die unter Formel I angegebenen Bedeutungen haben, mit Chlorsulfonylisocyanat umsetzt, aus der gebildeten Verbindung die Chlorsulfongruppierung reduktiv abspaltet, gewünschtenfalls die Hydroxyschutzgruppe $R^{1a}$ abspaltet, und gewünschtenfalls die gebildete Verbindung der Formel I gegebenenfalls von dem vorhandenen 3S,4S,1R'-Diastereomeren befreit.

EP 0 259 268 A1

**Beschreibung**

Verfahren zur Herstellung optisch aktiver β-Lactame

Die Erfindung betrifft ein neues vorteilhaftes, sterisch kontrolliertes Verfahren zur Herstellung optisch aktiver β-Lactamverbindungen, sowie neue Zwischenprodukte zur Herstellung dieser Verbindungen und Verfahren zu deren Herstellung.

Zur Herstellung von 3,4-disubstituierten β-Lactamen, die für die Synthese von antibiotisch wirksamen Penem- und/oder Carbapenemverbindungen benutzt werden können, sind bereits eine Reihe von Lösungsvorschlägen bekannt geworden. Voll zu befriedigen vermag allerdings keiner dieser Vorschläge, weil die industrielle Herstellung wegen zu zahlreicher Stufen, Bildung racemischer Produkte, niedrigen Ausbeuten, Nichtkristallinität der Zwischenprodukte oder daraus hergestellter Endprodukte, zu hohen Preisen der Ausgangsmaterialien und anderen Gründen immer noch erschwert ist.

In der Europäischen Patentanmeldung 167155 wird ein Verfahren zur Herstellung von (3R,4R,1'R)-3-(1-Hydroxyethyl)-4-acetoxyazetidinon, einem bekannten Zwischenprodukt für die Herstellung von Penemen und Carbapenemen, vorgeschlagen, in welchem an der Hydroxygruppe geschützter 3-Hydroxybutyraldehyd mit Halogentriniederalkylsilanen zum entsprechenden Silylenolether silyliert wird, der mit Chlorsulfonylisocyanat das entsprechende an der Hydroxygruppe geschützte 3-(1-Hydroxyethyl)-4-triniederalkylsilyloxy-1-chlorsulfonylazetidin-2-on ergibt, aus dem die Chlorsulfongruppe in der gleichen Stufe reduktiv abgespalten werden kann. Das erhaltene Azetidinon wird dann mit einem Halogentriniederalkylsilan behandelt und das erhaltene N-silylierte Produkt mit Acetanhydrid umgesetzt. Die Triniederalkylsilylschutzgruppe am N-1-Atom des Azetidinonringes wird hierauf mit Tetrabutylammoniumfluorid selektiv abgespalten. Das Verfahren verläuft relativ stereoselektiv. Ausgehend von dem 0-geschützten (3R)-3-Hydroxybutyraldehyd erhält man eine Mischung der trans- und cis-Form des Silylenolethers und nach der Reaktion mit Chlorsulfonylisocyanat hauptsächlich das (3R, 4R, 1'R)-Azetidinon neben etwa 10 % (3S,4S,1'R)-Azetidinon.

In der Europäischen Patentschrift 171064 wird eine Synthese von 3-(1-Hydroxyethyl)-4-acetoxyazetidinonen vorgeschlagen durch Anlagerung von (3R)-3-Hydroxybuttersäurederivaten an 1-Silylimin-2-propinverbindungen, nachfolgender Anlagerung von Wasser an das erhaltene 4-Ethinylazetidinon und Bayer-Villiger Umlagerung des erhaltenen 4-Acetylazetidinons. Das Verfahren hat den Nachteil, dass Gemische von 3,4-cis/trans-disubstituierten Azetidinonen gebildet werden, mit einem relativ ungünstigen Anteil an dem gewünschten Isomeren mit β-ständigem Wasserstoff am C-3-Verhältnis. Die Isomerisierung zu den gewünschten 3R,4R,1'R-Azetidinonen verläuft nicht einheitlich.

Als drittes Beispiel kürzlich veröffentlichter Azetidinonsynthesen wird die Europäische Patentanmeldung 179318 genannt. In mehreren Stufen wird aus 3-Butin-2-ol ein racemisches Z-3-Silyloxy-1-butenylphenylsulfid hergestellt, das mit Chlorsulfonylisocyanat zum racemischen (3S,4R,1'S)-3-(1-Silyloxyethyl)-4-phenylthioazetidinon zyklisiert wird. Die l'S-Konfiguration am C-1' der Seitenkette, die eine nachträgliche Isomerisierung bedingt, sowie die notwendige Racematspaltung der erhaltenen Azetidinone und die notwendige Oxidation der Sulfidgruppe bedeuten einen grossen Ausbeuteverlust.

3,4-Disubstituierte β-Lactame und Verfahren zu ihrer Herstellung sind ferner aus EP 82113, DE-OS 3 224 055, DE-OS 3 013 997 oder durch S. Hanessian et al., J. Am. Chem. Soc. 1985, 107, 1438-1439 bekannt.

Aufgabe der vorliegenden Erfindung ist ein neues vorteilhaftes, enantio- und diastereoselektives Verfahren zur Herstellung optisch aktiver β-Lactamverbindungen mit einer gegebenenfalls geschützten Hydroxyethylgruppe in 3-Stellung und einer veresterten Hydroxygruppe in 4-Stellung des β-Lactamringes zur Verfügung zu stellen, wobei bevorzugt die sterische Konfiguration 3R,4R,1'R erhalten wird. Eine weitere Aufgabe betrifft die Zurverfügungstellung von neuen Butenolestern und Verfahren zu deren Herstellung, die in dem genannten Verfahren als Ausgangsmaterialien benötigt werden.

Die Erfindung betrifft ein Verfahren zur Herstellung von (3R,4R,1'R)-β-Lactamverbindungen der Formel

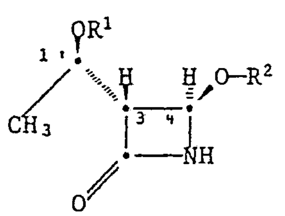

(I),

gegebenenfalls im Gemisch mit dem (3S,4S,1'R)-Diastereomeren, worin $R^1$ Wasserstoff oder eine unter den Ringschlussbedingungen nicht abspaltbare Hydroxyschutzgruppe $R^{1a}$ und $R^2$ den Acylrest einer schwachen Carbonsäure darstellt, dadurch gekennzeichnet, dass man eine Verbindung der Formel

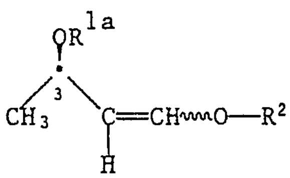

(II),

worin $R^{1a}$ und $R^2$ die unter Formel I angegebenen Bedeutungen haben, mit Chlorsulfonylisocyanat umsetzt, aus dem gebildeten Produkt die Chlorsulfongruppierung reduktiv abspaltet, gewünschtenfalls die Hydroxyschutzgruppe $R^{1a}$ abspaltet, und gewünschtenfalls die gebildete Verbindung der Formel I von dem vorhandenen 3S,4S,1'R-Diastereomeren befreit.

Eine unter den Ringschlussbedingungen nicht abspaltbare Hydroxyschutzgruppe $R^{1a}$ ist eine in der Penemchemie übliche Hydroxyschutzgruppe, z.B. ein üblicher Acyl-, substituierter Niederalkyl- oder Silylrest.

Geeignete Acylreste sind z.B. gegebenenfalls durch Halogen in 2-Stellung substituiertes Niederalkanoyl, z.B. Acetyl, Chloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromethoxycarbonyl oder 2,2,2-Trichlorethoxycarbonyl, Niederalkenyloxycarbonyl, z.B. Allyloxycarbonyl, oder gegebenenfalls durch Nitro substituiertes Phenylniederalkoxycarbonyl, z.B. 4-Nitrobenzyloxycarbonyl. Geeignete substituierte Niederalkylreste sind beispielsweise 2-Halogenniederalkylgruppen, z.B. 2-Chlor-, 2-Brom-, 2-Jod- und 2,2,2-Trichlorethyl, und gegebenenfalls durch Halogen, z.B. Chlor, Niederalkoxy, z.B. Methoxy, und/oder Nitro substituiertes Phenylniederalkyl, wie entsprechendes Benzyl. Besonders geeignete Hydroxyschutzgruppen sind z.B. trisubstituiertes Silyl, wie Triniederalkylsilyl, z.B. Triisopropylsilyl, 2,3-Dimethylbut-2-yl-dimethylsilyl oder tert.-Butyl-dimethylsilyl.

$R^2$ als Acylrest einer schwachen Carbonsäure ist beispielsweise Triniederalkylacetyl, wie Pivaloyl, 2,2-Dimethylpropionyl, oder Triethylacetyl, oder unsubstituiertes oder durch Substituenten 1. Ordnung ein- oder mehrfach, z.B. zwei- oder dreifach substituiertes Benzoyl, wie 4-Methoxy-, 2,4- oder 3,4-Dimethoxy- oder 3,4,5- oder 2,4,6-Trimethoxybenzoyl oder 4-Dimethylaminobenzoyl. $R^2$ kann auch der Acylrest eines Kohlensäurehalbesters -CO-O-R, oder einer substituierten Carbaminsäure -CO-NRR sein, worin R Nieder alkyl, wie Methyl oder Ethyl, Aryl, wie Phenyl, oder Arylniederalkyl, wie Benzyl darstellt. Bevorzugt ist $R^2$ Pivaloyl und 3,4-Dimethoxybenzoyl.

Die Umsetzung mit dem Chlorsulfonylisocyanat findet in einem inerten, d.h. an der Reaktion nicht teilnehmenden, Lösungsmittel statt, z.B. in Acetonitril, einem gegebenenfalls durch Halogen, wie Chlor, Niederalkoxy, wie Methoxy oder Ethoxy, oder Nitro ein oder mehrfach substituiertem Niederalkan, z.B. n-Hexan, Dimethoxyethan, Diethylether, Dichlormethan oder Nitromethan, einem heterocyclischen Lösungsmittel, wie Dioxan oder Tetrahydrofuran, Benzol, einem durch Halogen, wie Chlor oder Fluor, Niederalkoxy, wie Methoxy, Niederalkyl, wie Methyl, ein oder mehrfach, z.B. 2- bis 6-fach substituiertem Benzol, z.B. Nitrobenzol, Dinitrobenzol, Hexafluorbenzol, Hexamethoxybenzol oder Toluol, oder dergleichen, oder Mischungen davon, bei Temperaturen zwischen etwa -70°C und Raumtemperatur. Die Reaktionspartner werden etwa im molaren Verhältnis 1:1, oder mit einem kleinen Ueberschuss an Chlorsulfonylisocyanat verwendet.

Die nach Beendigung der Cycloaddition erhaltene Reaktionslösung enthält als Produkt das entsprechende 1-Chlorsulfonylazetidinon, d.h. ein Gemisch einer Verbindung der Formel I und Ia, im Verhältnis von >10:1, in denen die 1-Stellung durch die Chlorsulfonylgruppe besetzt ist. Das Produkt wird mit einem Reduktionsmittel versetzt, das die Chlorsulfonylgruppe reduktiv entfernt und durch Wasserstoff ersetzt. Geeignete Reduktionsmittel sind beisielsweise Metallhydride, wie Lithium-aluminiumhydrid, Natrium-bis(2-methoxyethoxy)-aluminiumhydrid oder Natriumborhydrid, oder Raney Nickel, und dergleichen, oder Mercaptane, z.B. Thiophenol. Die Reduktion wird gegebenenfalls unter vorheriger Verdünnung der Reaktionslösung mit einem der genannten Lösungsmittel, wenen Metallhydride benutzt werden mit Tetrahydrofuran, Toluol oder Ethylether, durchgeführt. Die Reduktionstemperatur liegt ebenfalls zwischen etwa -70°C und Raumtemperatur.

In verfahrensgemäss erhältlichen Verbindungen der Formel I, worin $R^1$ eine unter den Ringschlussbedingungen nicht abspaltbare Hydroxyschutzgruppe $R^{1a}$ bedeutet, kann, zur Herstellung von Verbindungen der Formel I, worin $R^1$ Wasserstoff bedeutet, die Hydroxyschutzgruppe $R^{1a}$ abgespalten und durch Wasserstoff ersetzt werden. Die Abspaltung erfolgt auf an sich bekannte Weise, je nach Art der Schutzgruppe, z.B. mittels Solvolyse oder Reduktion.

Beispielsweise kann 2-Halogenniederalkoxycarbonyl (gegebenenfalls nach Umwandlung einer 2-Bromniederalkoxycarbonylgruppe in eine 2-Jodniederalkoxycarbonylgruppe) und 4-Nitrobenzyloxycarbonyl durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators oder durch Behandeln mit einem Alkalimetall-, z.B. Natriumdithionit abgespalten werden. Gegebenenfalls substituiertes Benzyloxycarbonyl kann z.B. mittels Hydrogenolyse d.h. durch Behandeln mit Wasserstoff in Gegenwart eines geeigneten Hydrierkatalysators, wie eines Palladiumkatalysators, und Allyloxycarbonyl durch Umsetzen mit einer Verbindung des Palladiums, z.B. Tetrakis(triphenylphosphin)-palladium, gegebenenfalls in Gegenwart von Triphenylphosphin und in Gegenwart eines Allylgruppenakzeptors, wie einer Carbonsäure, z.B. 2-Ethylhexansäure, oder eines Salzes davon, oder Tributylzinnhydrid oder Dimedon,

abgespalten werden. 2-Halogenniederalkanoyl, z.B. 2-Chloracetyl, kann durch Behandeln mit Thioharnstoff in Gegenwart einer Base oder mit einem Salz, wie einem Alkalimetall-Salz, des Thioharnstoffs und anschliessender Solvolyse, wie Alkoholyse oder Hydrolyse, des entstandenen Kondensationsproduktes freigesetzt werden. Trisubstituierte Silylgruppen können durch Behandeln mit wässriger Fluorwasserstoffsäure in Acetonitril oder einem Fluoridanionen liefernden Salz davon, wie einem Alkalimetallfluorid, z.B. Natriumfluorid, in Anwesenheit eines makrocyclischen Polyethers ("Kronenether") oder mit einem Fluorid einer organischen quaternären Base, wie Tetraniederalkylammoniumfluorid, z.B. Tetrabutyl- oder Tetraethylammoniumfluorid (als Hydrate), abgespalten werden.

Die Verbindung der Formel I hat die gewünschte (3R,4R,1'R)-Konfiguration. Das gleichzeitig zu weniger als 10% entstehende (3S,4S,1'R)-Diasteromere hat die Formel

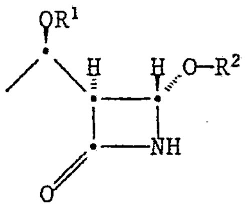

(Ia).

Die Abtrennung des unerwünschten (3S,4S,1'R)-Azetidinons kann vor oder nach Abspaltung der Hydroxyschutzgruppe $R^{1a}$ erfolgen, wird der bevorzugt auf einer späteren Stufe vorgenommen. Die Abtrennung des Stereoisomeren, bzw. die Reinigung der Verbindung der Formel I, erfolgt nach an sich bekannten Methoden, z.B. durch Destillation, Kristallisation und/oder Chromatographie und dergleichen.

Die Ausgangsverbindungen der Formel II sind neu und ebenfalls Gegenstand der vorliegendne Erfindung. Sie können als cis-Isomere der Formel

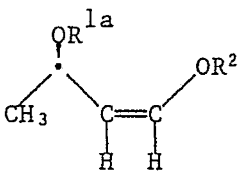

(IIa)

oder als trans-Isomere der Formel

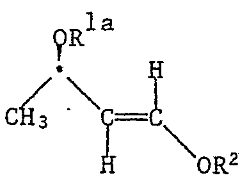

(IIb)

oder als cis/trans-Isomerengemisch (Formel II) vorliegen.

Sie werden erhalten, indem man einen Butyraldehyd der Formel

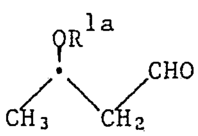

(III)

mit einer den Acylrest einer Carbonsäure der Formel

$HO-R^2$ (IV)

einführenden Mittel verestert und gewünschtenfalls das trans (E)-Isomer) aus dem erhältlichen Gemisch des trans (E)- und cis (Z)-Isomeren isoliert.

Ein den Acylrest $R^2$ einführendes Mittel ist entweder die Carbonsäure der Formel IV selbst in Gegenwart eines Kondensationsmittels oder ein reaktionsfähiges, funktionelles Derivat oder Salz dieser Carbonsäure.

Geeignete Kondensationsmittel sind bekannt und sind z.B. Carbodiimide, wie Diethyl-, Dipropyl, Dicyclohexyl- oder N-Ethyl-N'-3-dimethylaminopropylcarbodiimid, Carbonyldiimidazol, oder 1,2-Oxazoliumverbindungen, wie 2-Ethyl-5-phenyl-1,2-oxazolium-3'-sulfonat oder 2-tert.-Butyl-5-methyl-1,2-oxazoliumperchlorat, oder geeignete Acylaminoverbindung, z.B. 2-Ethoxy-1-ethoxycarbonyl-1,2-dihydrochinolin.

Die Kondensationsreaktion wird auf an sich bekannte Weise durchgeführt, vorzugsweise in Gegenwart

eines inerten, aprotischen Lösungsmittels, z.B. Methylenchlorid, Dimethylformamid, Acetonitril oder Tetrahydrofuran, gegebenenfalls unter Kühlen oder Erwärmen, z.B. in einem Temperaturbereich von etwa -40° C bis etwa +100° C, bevorzugt von etwa -20° C bis etwa +50° C, und gegebenenfalls unter Inertgas-, z.B. Stickstoffatomosphäre.

Reaktionsfähige, funktionelle Derivate einer Carbonsäure der Formel IV, die zur Bildung der Enolester-Funktion befähigt sind, sind bekannt. Entsprechende Derivate sind Anhydride der Carbonsäure der Formel IV, vorzugsweise gemischte Anhydride, z.B. mit einer anorganischen Säure, wie das Carbonsäurechlorid oder -bromid, das Carbonsäureazid, gemischte Anhydridride mit Phosphor-haltigen Säuren, z.B. Phosphorsäure, Diethylphosphorsäure, Schwefel-haltigen Säuren, z.B. Schwefelsäure, oder Cyanwasserstoffsäure, oder mit einer organischen Carbonsäure, z.B. mit einer unsubstituierten oder durch Halogen, z.B. Fluor oder Chlor, substituierten Niederalkancarbonsäure, z.B. Pivalinsäure oder Trifluoressigsäure, mit einem Niederalkylhalbester der Kohlensäure, z.B. dem Ethyl- oder Isobutylhalbester der Kohlensäure, oder mit einer organischen, z.B. aliphatischen oder aromatischen, Sulfonsäure, z.B. Methansulfonsäure oder p-Toluolsulfonsäure. Reaktionsfähige funktionelle Derivate einer Carbonsäure der Formel IV sind ferner aktivierte Ester, die beispielsweise durch Kondensation mit einem vinylogen Alkohol, d.h. mit einem Enol, z.B. einem vinylogen Niederalkenol, gebildet werden, Iminomethylesterhalogenide, z.B. Dimethyliminomethylesterchlorid, hergestellt aus der Carbonsäure der Formel IV und z.B. Dimethyl-(1-chlorethyliden)-iminiumchlorid der Formel $[(CH_3)_2N^+=C(Cl)CH_3]Cl^-$, Arylester, z.B. ein durch Halogen, z.B. Chlor, und/oder Nitro substituierter Phenylester, z.B. der Pentachlorphenyl-, 4-Nitrophenyl- oder 2,3-Dinitrophenylester, N-heteroaromatische Ester, z.B. der N-Bentriazolester, oder N-Diacyliminoester, z.B. der N-Succinylimino- oder N-Phthalyliminoester.

Die Acylierung mit dem reaktionsfähigen funktionellen Derivat der Carbonsäure der Formel IV wird bevorzugt in Anwesenheit einer geeigneten Base, beispielsweise einem tertiären Amin, z.B. Triniederalkylamin, wie Trimethylamin, Triethylamin oder Ethyl-di-isopropylamin, einem N,N-Diniederalkylanilin, wie N,N-Dimethylanilin, einem N-niederalkylierten Morpholin, wie N-Methylmorpholin, einer Base vom Pyridin-Typ, z.B. Pyridin oder Chinolin, oder einer Diazabicyclobase, wie 1,5-Diazabicyclo[5.4.0]undec-5-en, oder einer anorganischen Base, beispielsweise einem Alkalimetall- oder Erd alkalimetallhydroxid, -carbonat oder -hydrogencarbonat, wie Natrium-, Kalium- oder Calciumhydroxid, -carbonat oder -hydrogencarbonat, vorzugsweise in einem inerten, insbesondere wasserfreien, aprotischen Lösungsmittel oder Lösungsmittelgemisch vorgenommen, beispielsweise in einem Carbonsäureamid, wie Dimethylformamid, einem halogenierten Kohlenwasserstoff, wie Methylenchlorid, Tetrachlorkohlenstoff oder Chlorbenzol, einem cyclischen Ether, wie Tetrahydrofuran oder Dioxan, einem Ester, wie Essigsäureethylester, oder einem Nitril, wie Acetonitril, oder in einer Mischung davon, gegebenenfalls, bei erniedrigter oder erhöhter Temperatur, z.B. in einem Temperaturbereich von etwa -40° C bis etwa +100° C, bevorzugt von etwa +50° C bis etwa 100° C, und gegebenenfalls unter Inertgas-, z.B. Stickstoffatmosphäre.

Die Acylierung einer Verbindung der Formel II kann auch in Gegenwart einer geeigneten Acylase erfolgen. Ein bei der Acylierungsreaktion eingesetztes, reaktionsfähiges funktionelles Derivat einer Säure der Formel IV kann auch in situ gebildet werden. Bei der Veresterung entsteht ein trans/cis-Gemisch der Verbindung der Formel II.

Je nach Art von $R^{1a}$, dem eingesetzten Derivat der Carbonsäure IV und den Reaktionsbedingungen können unterschiedliche Mengen des cis-Isomeren entstehen. Bei Verwendung von Silylschutzgruppen $R^{1a}$, insbesondere Dimethyl-(2,3-dimethylbut-2-yl)- oder Dimethyl-tert.butylsilyl, Pivalinsäurechlorid oder 3,4-Dimethoxybenzoesäurechlorid und Triethylamin in Dimethylformamid entstehen trans/cis-Gemische im Verhältnis von etwa 9:1.

Die Reinigung kann durch Destillation oder auch Chromatographie erfolgen. Die erhältliche trans-Verbindung der Formel IIb wird in reiner Form oder bevorzugt ohne Auftrennung im Gemisch mit dem cis-Isomeren der Formel IIa in die nächste Stufe eingesetzt.

Ueberraschenderweise werden nach dem folgenden Umsatz mit Chlorsulfonylisocyanat die beiden theoretisch möglichen diastereomeren 3,4-cis-Azetidinone der Formel

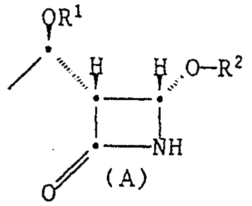

und

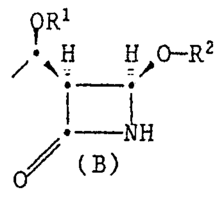

nicht gefunden.

Die Verfahren der vorliegenden Erfindung umfassen auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird, sowie die Kombination der beiden Verfahren. Ferner können Ausgangsstoffe in Form von Derivaten verwendet oder

in situ, gegebenenfalls unter den Reaktionsbedingungen, hergestellt werden.

Verbindungen der Formel I, die gegebenenfalls bis zu höchstens 10 % (3S,4S,1'R)-Diastereomere enthalten, sind wertvolle Zwischenprodukte, die auf an sich bekannte Weise zu antibiotisch wirksamen Penem- oder Carbapenemverbindungen weiterverarbeitet werden können. Die Weiterverarbeitung von Verbindungen der Formel I, worin $R^1$ eine Hydroxyschutzgruppe $R^{1a}$ ist, kann analog derjenigen von bekannten Azetidinonverbindungen der Formel I, z.B. wie von T. Hayashi et al., Chem. Pharm. Bull. 29, 3158, 1981 oder in den Europäischen Patentanmeldungen Nr. 42026 (für Peneme) oder Nr. 78026 (für Carbapeneme) beschrieben ist, erfolgen.

Die erfindungsgemäss erhältlichen Verbindungen der Formel I, worin $R^1$ Wasserstoff ist, die gegebenenfalls noch das (3S,4S,1'R)-Diastereomere enthalten, können gemäss EP 215739 in Penemverbindungen übergeführt werden. Diese Europäische Patentanmeldung betrifft ein verbessertes Verfahren zur Herstellung von Verbindungen der Formel

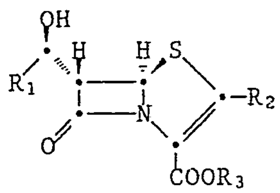

(I'),

oder Salzen davon, dadurch gekennzeichnet, dass man

a) eine Verbindung der Formel

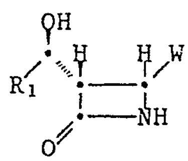

(II')

mit einer den Thiocarbonsäurerest der Formel

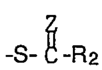

(III')

einführenden Verbindung behandelt,

b) eine erhältliche Verbindung der Formel

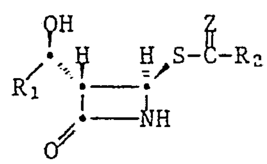

(IV')

mit einem den Acylrest eines Oxalsäurehalbesters der Formel

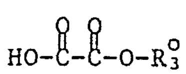

(V')

einführenden Acylierungsmittel behandelt,

c) eine erhältliche Verbindung der Formel

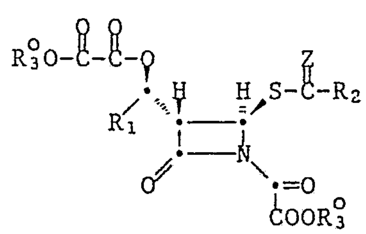

(VII')

mit einer organischen Verbindung des dreiwertigen Phosphors behandelt, und
d) in einer erhältlichen Verbindung der Formel

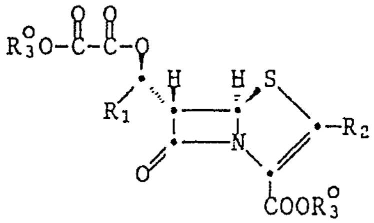

(VIII'),

die Oxalylestergruppe -CO-COO-$R_3^O$ und gewünschtenfalls die Schutzgruppe $R_3^O$ und/oder eine gegebenenfalls im Rest $R_2$ vorhandene Schutzgruppe $R_2^O$ in beliebiger Reihenfolge abspaltet und durch Wasserstoff ersetzt, und gewünschtenfalls eine erhältliche Verbindung der Formel I mit salzbildender Gruppe in ein Salz oder ein erhältliches Salz in die freie Verbindung überführt, in welchen Verbindungen $R_1$ Methyl, $R_2$ ein organischer Rest, der eine funktionelle Gruppe tragen kann, die gegebenenfalls durch eine übliche Schutzgruppe $R_2^O$ geschützt sein kann, $R_3$ Wasserstoff oder eine übliche Carboxylschutzgruppe $R_3^O$, W eine gegen einen Thiocarbonsäurerest der Formel III austauschbare Gruppe und Z Sauerstoff oder Schwefel darstellen.

Ein analoges Verfahren ist in EP 221846 beschrieben.

Die Abgangsgruppe W in den Verbindungen der Formel II' umfasst auch den Rest -$OR^2$ wie er in den Verbindungen der Formeln I und II der vorliegenden Anmeldung definiert ist. Die beschriebenen bevorzugten Acyloxy-Abgangsgruppen W, d.h. Acetoxy, Benzoyloxy, durch Nitro substituiertes Benzoyloxy und Phenylacetyloxy, sind jedoch für die vorliegende Erfindung weniger gut geeignet, indem damit der Azetidinonringschluss unbefriedigend verläuft. Andererseits lassen sich die vorliegenden Verbindungen der Formel I leicht zu den Verbindungen der Formel IV' thiolisieren, wobei die 1'-Hydroxygruppe auch geschützt sein kann. Die Verbindungen der Formel IV' werden in kristalliner Form erhalten und können dadurch leicht von dem unerwünschten (3R,4S,1'R)-Diastereomeren abgetrennt werden.

Die vorliegende Erfindung betrifft daher auch ein verbessertes Verfahren zur Herstellung von Verbindungen der Formel IV', worin die 1'-Hydroxygruppe gegebenenfalls durch die Gruppe $R^{1a}$ geschützt sein kann, dadurch gekennzeichnet, dass man eine Verbindung der Formel I, gegebenenfalls im Gemisch mit einer Verbindung der Formel Ia mit einer den Thiocarbonsäurerest der Formel -SC(=Z)-$R_2$ (III') einführenden Verbindung behandelt.

Den Rest der Formel III' einführende Verbindungen sind die entsprechenden Thiocarbonsäuren H-S-C(=Z)-$R_2$ selbst oder Salze davon, z.B. Alkalimetallsalze, wie das Natrium- oder Kaliumsalz.

Die Substitution kann in einem organischen Lösungsmittel, wie in einem Niederalkanol, z.B. Methanol oder Ethanol, einem Niederalkanon, z.B. Aceton, einem Niederalkancarbonsäureamid, z.B. Dimethylformamid, einem cyclischen Ether, z.B. Tetrahydrofuran oder Dioxan, oder in einem ähnlichen inerten Lösungsmittel durchgeführt werden. Die Reaktion wird üblicherweise bei Raumtemperatur durchgeführt, kann aber auch bei erhöhter oder erniedrigter Temperatur, z.B. bei etwa 0° bis etwa 40°C, durchgeführt werden.

Die eintretende Gruppe -S-C(=Z)-$R_2$ wird von dem Rest $R_1$-CH(OH)-ausschliesslich in die trans-Stellung dirigiert. Die Verbindungen der Formel IV' lassen sich leicht kristallisieren und dadurch von gegebenenfalls vorhandenen, aus Verbindungen der Formel Ia gebildeten, (3R,4S,1'R)-Diastereomeren befreien.

Die vorliegende Erfindung betrifft auch die neuen Verbindungen der Formel I, gegebenenfalls im Gemisch mit dem (3S,4S,1'R)-Diastereomeren der Formel Ia. Im Gemisch liegen die Verbindungen I zu Ia im Verhältnis $>10:1$ vor.

Die Erfindung betrifft auch die Verwendung einer Verbindung der Formel I, gegebenenfalls im Gemisch mit dem (3S,4S,1'R)-Diastereomeren (Ia) in einem Verfahren zur Herstellung von Verbindungen der Formel

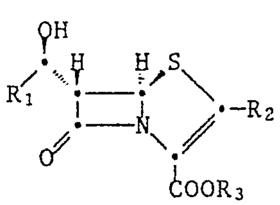

(I'),

oder Salzen davon, dadurch gekennzeichnet, dass man
a) eine Verbindung der Formel I, gegebenenfalls im Gemisch mit einer Verbindung der Formel Ia, mit einer den Thiocarbonsäurerest der Formel

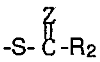 (III')

einführenden Verbindung behandelt,

b) eine erhältliche Verbindung der Formel

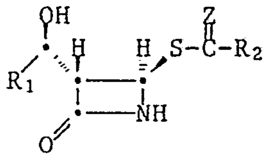 (IV')

mit einem den Acylrest eines Oxalsäurehalbesters der Formel

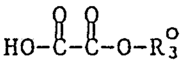 (V')

einführenden Acylierungsmittel behandelt,

c) eine erhältliche Verbindung der Formel

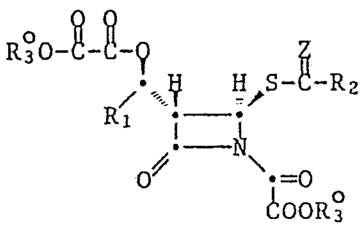 (VII')

mit einer organischen Verbindung des dreiwertigen Phosphors behandelt, und

d) in einer erhältlichen Verbindung der Formel

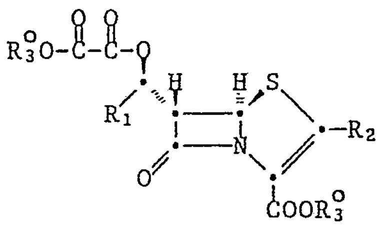 (VIII'),

die Oxalylestergruppe -CO-COO-$R_3^O$ und gewünschtenfalls die Schutzgruppe $R_3^O$ und/oder eine gegebenenfalls im Rest $R_2$ vorhandene Schutzgruppe $R_2^O$ in beliebiger Reihenfolge abspaltet und durch Wasserstoff ersetzt, und gewünschtenfalls eine erhältliche Ver bindung der Formel I mit salzbildender Gruppe in ein Salz oder ein erhältliches Salz in die freie Verbindung überführt, in welchen Verbindungen, $R_1$ Methyl, $R_2$ ein organischer Rest, der eine funktionelle Gruppe tragen kann, die gegebenenfalls durch eine übliche Schutzgruppe $R_2^O$ geschützt sein kann, $R_3$ Wasserstoff oder eine übliche Carboxylschutzgruppe $R_3^O$, und Z Sauerstoff oder Schwefel darstellen.

Die folgenden Beispiele dienen zur Illustration der Erfindung, sind aber nicht als eine Limitierung des Umfanges aufzufassen.

Beispiel 1: (3R)-3-[Dimethyl(2,3-dimethylbut-2-yl)silyloxy]but-1-en-1-yl-pivaloat

Zu einer Lösung von 18,7 g (81,3 mmol) (3R)-3-[Dimethyl(2,3-dimethylbut-2-yl)silyloxy]butyraldehyd in 34,5 ml Dimethylformamid und 41,1 ml (295 mmol) Triethylamin werden 25,8 ml (210 mmol) Pivalinsäurechlorid gegeben. Die Reaktionsmischung wird unter Stickstoffatmosphäre bei 90-100°C Badtemperatur 20$^1/_2$ Stunden gerührt. Die dunkelbraune Suspension wird mit 150 ml n-Hexan und 60 ml Wasser aufgearbeitet. Die organische Phase wird über Natriumsulfat getrocknet, am Vakuum eingedampft und der schwarze Rückstand durch Hochvakuumdestillation über eine kurze Vigreux-Kolonne gereinigt, Sdp. 96-100°C/0,1 Torr. Die Titelverbindung wird als klare, gelbe Flüssigkeit, gemäss 360 MHz-$^1$H-NMR-Spektrum als Gemisch von E/Z-Isomeren im Verhältnis von 88:12, erhalten.

IR ($CH_2Cl_2$) 2970, 2880, 1740, 1140. $^1$H-NMR (60 MHz, $CDCl_3$) der Hauptkomponente zeigt Signale u.a. bei 7,2 ppm (1H, d, J = 12 Hz); 5,4 ppm (1H d x d, $J_1$ = 12 Hz, $J_2$ = 6 Hz), 4,3 ppm (1H 5-Linien System).

Das Ausgangsmaterial kann wie folgt hergestellt werden:

(3R)-3-[Dimethyl(2,3-dimethylbut-2-yl)silyloxy]buttersäureethylester

Eine Lösung von 27,9 g (211,2 mmol) (3R)-3-Hydroxybuttersäureethylester in 21 ml Dimethylformamid wird mit 25,8 g (379 mmol) Imidazol und 45 g (253,5 mmol) Dimethyl-(2,3-dimethylbut-2-yl)-chlorsilan versetzt. Unter anfänglicher Kühlung mit Eis/Wasser wird die Reaktionstemperatur bei 20°C gehalten und anschliessend während 18 Stunden bei Raumtemperatur gerührt. Zur Aufarbeitung wird die Reaktionsmischung mit 250 ml Essigsäureethylester und 50 ml Wasser versetzt. Nach Abtrennen der organischen Phase wird diese noch dreimal mit je 50 ml Wasser gewaschen, über Natriumsulfat getrocknet und im Vakuum eingedampft. Das Rohprodukt wird durch Hochvakuumdestillation gereinigt, Sdp. 60°C/0,05 Torr. Die Titelverbindung wird als farblose Flüssigkeit erhalten.

$[\alpha]_D^{20}$ = -23° (c = 0,6, $CHCl_3$). IR ($CH_2Cl_2$) 2960, 2870, 1730, 1375, 1300, 1185, 1000, 835, 820.

(3R)-3-[Dimethyl(2,3-dimethylbut-2-yl)silyloxy]butyraldehyd

Unter Stickstoffatmosphäre werden 30,9 g (112,7 mmol) (3R)-3-[Dimethyl(2,3-dimethylbut-2-yl)silyloxy]buttersäureethylester in 140 ml n-Hexan gelöst. Bei -70°C Innentemperatur werden anschliessend 113,5 ml 1 molare Diisobutylaluminiumhydrid-Lösung in Hexan so zugetropft, dass die Innentemperatur nie über -50°C steigt. Nach 45 Minuten Rühren bei -70°C werden tropfenweise 23 ml abs. Ethylalkohol und anschliessend 46 ml Ethylalkohol/Wasser 1:1 bei -60 bis -70°C zugetropft. Die Reaktionsmasse wird während 1 1/2 Stunden auf Raumtemperatur aufgewärmt, mit 200 g Natriumsulfat versetzt dund weitere 10 Minuten gerührt. Nach zweimaliger Filtration über Hyflo und Natriumsulfat wird das klare Filtrat am Vakuum eingedampft und das Rohprodukt durch Hochvakuumdestillation über eine Kurze Vigreux-Kolonne gereinigt, Sdp. 69°C/0,3 Torr. Die Titelverbindung wird als farblose Flüssigkeit erhalten.

$[\alpha]_D^{20}$ = -9,0° (c = 0,5, $CHCl_3$). IR ($CH_2Cl_2$) 2970, 2880, 1730, 1390, 1030, 840.

Beispiel 2: (3R)-3-[Dimethyl(2,3-dimethylbut-2-yl)silyloxy]but-1-en-1-yl-3,4-dimethoxybenzoat

Zu einer Lösung von 3,2 g (13,9 mmol) (3R)-3-[Dimethyl(2,3-dimethylbut-2-yl)silyloxy]butyraldehyd in 15 g Dimethylformamid und 5,2 g (51,5 mmol) Triethylamin werden 6,8 g (34 mmol) 3,4-Dimethoxybenzoesäurechlorid gegeben und anschliessend 17 Stunden bei 95°C Badtemperatur gerührt. Zur Aufarbeitung wird mit 50 ml Hexan und 50 ml Wasser versetzt, die organische Phase abgetrennt und noch 4 x mit je 20 ml Wasser gewaschen, über Natriumsulfat getrocknet und am Vakuum eingedampft. Der Rückstand wird an 250 g Kieselgel mit Toluol/Essigester 9:1 chromatographisch gereinigt, wobei die Titelverbindung als gelbbraunes Oel erhalten wird.

$R_f$-Wert 0,65 (Merck Fertigplatten), Toluol/Essigester 1:1, Iod als Entwicklungsreagens. Laut 360 MHz-$^1$H-NMR-Specktrum liegt ein Gemisch von E/Z-Isomeren im Verhältnis von 85:15 vor. IR ($CH_2Cl_2$) 2960, 2870, 2840, 1720, 1600, 1510, 1220. $^1$H-NMR (60 MHz, $CDCl_3$) der Hauptkomponente zeigt Signale u.a. bei 5,56 ppm (1H, d x d, $J_1$ = 12 Hz, $J_2$ = 6 Hz), 4,4 ppm (1H, 5-Linien System), 3,86 ppm (6H, s).

Beispiel 3: (3R, 4R, 1'R)-3-{1-[Dimethyl(2,3-dimethylbut-2-yl)silyloxy]ethyl}-4-pivaloyloxyazetidin-2-on

Eine Lösung von 15,7 g (50 mmol) (3R)-3-[Dimethyl(2,3-dimethylbut-2-yl)silyloxy]but-1-en-1-yl-pivaloat (E:Z = 88:12) in 150 ml Ether unter Argonatmosphäre wird auf -60°C gekühlt, unter gutem Rühren in zwei Portionen im Einstundenabstand je innert 15 Minuten eine Lösung von total 10 ml (114 mmol) Chlorsulfonylisocyanat in 10 ml Toluol zugetropft und anschliessend 19 Stunden bei +4°C reagieren gelassen. Danach wird auf -50 bis -55°C gekühlt, nacheinander mit 200 ml Toluol und während 30 Minuten unter gutem Rühren vorsichtig mit einer Lösung von 31 ml 3,5 molarem Natriumdihydro-bis-(2-methoxyethoxy)-aluminat in 300 ml Toluol versetzt und eine Stunde bei -50°C ausreagieren gelassen. Die Reaktionsmischung wird zur Aufarbeitung auf eine eiskalte Mischung von 1,25 l wässriger 1 molarer Kaliumnatriumtartrat-Lösung und 1,25 l Toluol gegossen und 20 Minuten heftig gerührt. Nach Filtration über Hyflo wird die organische Phase abgetrennt, mit Sole gewaschen, über Natriumsulfat getrocknet und am Vakuum eingeengt. Der Rückstand wird an 1,5 kg Kieselgel mit zuerst 10 l Toluol/Essigester 95:5 und anschliessend 5 l Toluol/ Essigester 4:1 chromatographisch gereinigt. Es werden 1 l Fraktionen gesammelt. Die Fraktionen 7-10 enthalten die Titelverbindung als >10:1 Gemisch von Diastereomeren mit (3R, 4R, 1'R)-respektive (3S, 4S, 1'R)-Konfiguration.

$[\alpha]_D^{20}$ = +36° (c = 0,5, $CHCl_3$), Smp. 46-50°C, IR ($CH_2Cl_2$) 3400, 2950, 2860, 1785, 1725, 1130. Charakteristische Signale im 360 MHz-$^1$H-NMR ($CDCl_3$) vom (3R, 4R, 1'R)-Isomeren liegen bei 5,79 ppm (1H, d, J = 1,5 Hz), 3,19 ppm (1H, d x d, $J_1$ = 3,9 Hz, $J_2$ = 1,5 Hz), 1,26 ppm (3H, d, J = 7 Hz).

Durch nochmalige Chromatographie und zweimalige Kristallisation aus Hexan bei -50° kann die Titelverbindung in reiner Form erhalten werden, Smp. 70-72°C, $[\alpha]_D^{20}$ = +47° (c = 0,5, $CHCl_3$).

Beispiel 4: (3R, 4R, 1'R)-3-{1-[Dimethyl(2,3-dimethylbut-2-yl)silyloxy]ethyl}-4-(3,4-dimethoxybenzoyloxy)azetidin-2-on

Eine Lösung von 2,0 g (5 mmol) (3R)-3-[Dimethyl(2,3-dimethylbut-2-yl)silyloxy]but-1-en-1-yl-3,4-dimethoxybenzoat (E:Z = 85:15) in 15 ml Ether wird unter Argonatmosphäre auf -60°C gekühlt. Unter gutem Rühren wird dazu eine Lösung von 1 ml (11,5 mmol) Chlorsulfonylisocyanat in 1 ml Toluol getropft und anschliessend 20 Stunden bei +4°C reagieren gelassen. Danach wird auf -60°C abgekühlt, 20 ml Toluol zugegeben,

vorsichtig eine Lösung von 3,1 ml 3,5 molarer Natriumdihydro-bis-(2-methoxyethoxy)-aluminat in 30 ml Toluol zugetropft und eine Stunde bei -50°C ausreagieren gelassen. Die Reaktionsmischung wird zur Aufarbeitung auf eine eiskalte Mischung von 250 ml Toluol und 250 ml wässriger 0,1 molare Kaliumnatriumtartrat-Lösung gegossen und 15 Minuten heftig gerührt. Nach anschliessender Filtration über Hyflo wird die organische Phase abgetrennt, mit Sole gewaschen, über Natriumsulfat getrocknet und am Vakuum eingeengt. Der Rückstand wird an 150 g Kieselgel mit Toluol/ Essigester 9:1 chromatographisch gereinigt. Die Fraktionen 28-36 enthalten die viskose Titelverbindung im >10:1 Gemisch mit dem Diastereomeren mit (3S, 4S, 1'R)-Konfiguration.

$[\alpha]_D^{20}$ = +48° (C = 0,5, $CHCl_3$). IR ($CH_2Cl_2$), 3410, 2960, 2870, 2840, 1785, 1710, 1600, 1175, 1135, 1100, 1025. Charakteristische Signale im 360-MHz-$^1$H-NMR ($CDCl_3$) vom (3R, 4R, 1'R)-Isomeren: 6,07 ppm (1H, d, J = 1,5 Hz); 3,34 ppm (1H, d x d, $J_1$ = 3,9 Hz, $J_2$ = 1,5 Hz); 1,32 (3H, d, J = 6,5 Hz).

Beispiel 5: (3S, 4R, 1'R)-3-{1-[Dimethyl(2,3-dimethylbut-2-yl)silyloxy]ethyl}-4-(N-cinnamyloxycarbonylglycylthio)azetidin-2-on

Eine Lösung von 226,3 mg (0,9 mmol) N-Cinnamyloxycarbonylthioglycin in 0,9 ml wässriger 1N NaOH wird mit 0,1 N wässriger HCl auf pH 8 gestellt und bei 25°C unter Stickstoffatmosphäre zu 160,8 mg (0,45 mmol) (3R, 4R, 1'R)-3-{1-[Dimethyl(2,3-dimethylbut-2-yl)silyloxy]ethyl}-4-pivaloyloxyazetidin-2-on (>10:1 Gemisch vom Beispiel 3) in 0,75 ml Acetonitril getropft. Nach Zugabe von 1 Tropfen 0,1 N wässriger NaOH wird 20 Minuten bei 25°C gerührt. Zur Aufarbeitung werden 20 ml Essigsäureethylester und 10 ml Sole mit dem Reaktions gemisch geschüttelt. Die organische Phase wird abgetrennt, mit 5 ml 5%iger wässriger $NaHCO_3$-Lösung und zweimal mit 5 ml Sole gewaschen, über Natriumsulfat getrocknet und am Rotationsverdampfer vom Lösungsmittel befreit. Der Rückstand wird sofort an 6 g Kieselgel mit Toluol/ Essigsäurethylester 9:1 bis 4:1 chromatographisch gereinigt. Man erhält die Titelverbindung vom Smp. 98-100°C (Petrolether/$CH_2Cl_2$).

$[\alpha]_D^{20}$ = +87° (c = 0,5, $CHCl_3$). IR ($CH_2Cl_2$) 3440, 3400, 2960, 1775, 1730, 1695, 1505, 965, 945.

Beispiel 6: (3R, 4R, 1'R)-3-(1-Hydroxyethyl)-4-pivaloyloxyazetidin-2-on

Zu einer Lösung von 285,6 mg (0,8 mmol) (3R, 4R, 1'R)-3-{1-[Dimethyl(2,3-dimethylbut-2-yl)silyloxy]ethyl}-4-pivaloyloxyazetidin-2-on in 10 ml Acetonitril werden bei Raumtemperatur 4 ml wässrige 48%ige Fluorwasserstoffsäure gegeben. Nach 1 Stunde Rühren wird die Reaktionsmischung mit 150 ml Methylenchlorid verdünnt und mit 8 g Natriumbicarbonat neutralisiert. Nach Zugabe von 10 g Natriumsulfat wird filtriert und das Filtrat am Vakuum eingeengt. Der Rückstand wird an 6 g Kieselgel mit Toluol/Essigester 1:1 chromatographisch gereinigt, wobei die Titelverbindung erhalten wird. Smp. 131-133°C ($CH_2Cl_2$/Petrolether).

$[\alpha]_D^{20}$ = +48° (c = 0,5, $CHCl_3$). IR ($CH_2Cl_2$) 3400, 2950, 2920, 2860, 1780, 1725, 1135.

Beispiel 7: (3S, 4R, 1'R)-3-(1-Hydroxyethyl)-4-(N-cinnamyloxycarbonylglycylthio)azetidin-2-on

Eine Lösung von 136 mg (0,54 mmol) N-Cinnamyloxycarbonylthioglycin in 0,54 ml 1N wässriger Natronlauge wird mit 0,1N wässriger HCl auf pH 8 gestellt und bei 25°C unter Stickstoffatmosphäre zu 98 mg (0,45 mmol) (3R, 4R, 1'R)-3-(1-Hydroxyethyl)-4-pivaloyloxyazetidin-2-on in 0,9 ml Acetonitril gegeben. Nach Zugabe von 1 Tropfen 0,1N wässriger Natronlauge wird 20 wird 20 Minuten gerührt und dann wie im Beispiel 5 aufgearbeitet. Nach chromatographischer Reinigung des Rohproduktes über Kieselgel mit Toluol/Essigester 3:2 wird die Titelverbindung erhalten. Smp. 100-101°C (Diisopropylether/$CH_2Cl_2$).

$[\alpha]_D^{20}$ = +112° (c = 0,42, $CHCl_3$). IR ($CH_2Cl_2$) 3440, 3400, 2970, 1775, 1730, 1700, 1510, 970.

Beispiel 8: (5R,6S,1'R)-6-(1'-Hydroxyethyl)-2-cinnamyloxycarbonylaminomethylpenem-3-carbonsäureallylester:

Eine Lösung von 310 mg (0,852 mmol) (3S,4R,1'R)-3-(1'-Hydroxyethyl)-4-(N-cinnamyloxycarbonylglycylthio)-2-azetidinon (Beispiel 7) in 3,2 ml Methylenchlorid wird unter Rühren und Feuchtigkeitsausschluss bei -10 bis -15° nacheinander mit 0,216 ml (1,76 mmol) frisch destilliertem Oxalsäureallylesterchlorid und 0,301 ml (1,75 mmol) N-Ethyl-diissopropylamin versetzt und 30 Min. bei -10° weiter gerührt. Eine IR-Probe ($CH_2Cl_2$) zeigt die charakteristische Oxalimid-Carbonyl-Absorption bei 1820 $cm^{-1}$. Zum Aufarbeiten wird das Reaktionsgemisch mit 2 ml $CH_2Cl_2$ verdünnt und zweimal mit je 4 ml wässriger Pufferlösung gewaschen. Die wässrigen Anteile werden einmal mit $CH_2Cl_2$ nachextrahiert. Die vereinigten organischen Phasen werden mit $Na_2SO_4$ getrocknet, im Vakuum eingedampft und das Rohprodukt am Hochvakuum getrocknet. Der erhaltene harzige Rückstand enthaltend den (3S,4R,1'R)-2-[4-(N-Cinnamyloxycarbonylglycylthio)-3-(1'-allyloxyoxalyloxyethyl)-2-oxo-1-azetidinyl]-2-oxoessigsäureallylester [IR ($CH_2Cl_2$) 3440 (NH);1820 (N-$\overset{O}{\overset{\|}{C}}$-$\overset{O}{\overset{\|}{C}}$); 1750, 1725 ($\overset{O}{\overset{\|}{C}}$)] wird in 0,37 ml Dioxan gelöst und unter $N_2$-Atmosphäre bei Raumtemperatur mit 0,51 ml (2,1 mmol) Triisopropylphosphit versetzt. Nach 15 Stunden bei Raumtemperatur kann im IR kein Oxalimid mehr festgestellt werden. (Bande bei 1820 $cm^{-1}$).

Das Reaktionsgemisch wird am Vakuum (Kugelrohr, 0,1 Torr) vom überschüssigen Phosphit und vom Phosphat befreit. Das zurückbleibende rohe Phosphoran wird zur Cyclisierung in 3,3 ml Dioxan gelöst und 12 Stunden bei 100° gerührt. Das Reaktionsgemisch, enthaltend den (5R,6S,1'R)-2-Cinnamyloxycarbonylaminomethyl-6-[1'-(allyloxyoxalyloxy)ethyl]-2-penem-3-carbonsäureethylester (für analytische Zwecke wird eine

Probe des rohen Cyclisierungsproduktes an Kieselgel mit Toluol / Essigsäureethylester 9:1 gereinigt; F. 87-88°C, UV (EtOH) 315 nm, IR ($CH_2Cl_2$) 3440 (NH), 1795, 1770, 1745, 1720 ( $\overset{O}{\overset{\|}{C}}$ ) wird im Vakuum bei Raumtemperatur eingedampft und der Rückstand bei 0° in 4 ml Methanol gelöst. Anschliessend werden 2 ml 5%-ige wässrige $NaHCO_3$-Lösung bei 0° zugetropft. Nach 15 Minuten werden 2 ml Wasser zugetropft und nach 30 Minuten wird die ausgefallene Titelverbindung abfiltriert und aus Methanol umkristallisiert; F. 178-180°.

Beispiel 9: (5R, 6S, 1'R)-2-Aminomethyl-6-(1'-hydroxyethyl)-2-penem-3-carbonsäure

Eine Lösung von 48 mg (0,108 mmol) (5R,6S,1'R)-6-)1'-Hydroxyethyl)-2-cinnamyloxycarbonylaminomethyl-2-penem-3-carbonsäureallylester und 22,3 mg (0,159 mmol) Dimedom in 1 ml Tetrahydrofuran wird während 5 Minuten mit Stickstoff gespült und unter Stickstoff-Atmosphäre bei Raumtemperatur mit 1,6 mg Tetrakistriphenylphosphin-palladium versetzt. Nach ca. 30 Minuten beginnt die Titelverbindung auszufallen. Nach weiteren 2,5 Stunden Rühren wird das Produkt abfiltriert, mit Tetrahydrofuran gut gewaschen und am Hockvakuum getrocknet.

Man erhält die Titelverbindung vom Rf 0,50 (DC $H_2O$, OPTI $UPC_{12}$); UV($H_2O$) 308 nm.

Beispiel 10: (3S, 4R, 1'R)-3-{1-[Dimethyl(2,3-dimethylbut-2-yl)silyloxy]ethyl}-4-(N-allyloxycarbonylglycylthio)azetidin-2-on

a) Eine Lösung von 191,3 mg (0,54 mmol) N-Allyloxycarbonylthioglycin-dicyclohexylammoniumsalz in 0,2 ml Wasser wird mit 0,54 ml wässriger 1N NaOH versetzt und zweimal mit 1 ml Methylenchlorid extrahiert. Die wässrige Phase wird mit 0,1N wässriger HCl auf pH 7-8 gestellt. Die erhaltene wässrige Thiolsäurelösung wird bei 25°C zu einer Lösung von 160,8 mg (0,45 mmol) (3R, 4R, 1'R)-3-{1-[Dimethyl(2,3-dimethylbut-2-yl)silyloxy]ethyl}-4-pivaloyloxy-azetidin-2-on (>10:1 Gemisch vom Beispiel 3) in 0,75 ml Acetonitril unter Stickstoffatmosphäre gegeben. Es wird noch 1 Tropfen 0,1N wässrige NaOH zugegeben und 45 Minuten bei 25°C gerührt. Zur Aufarbeitung werden in einem Scheidetrichter 15 ml Essigsäureethylester vorgelegt und das Reaktionsgemisch dazu gegeben. Nach gute Schütteln und Abtrennen der organischen Phase wird diese einmal mit 5 ml 5%iger wässriger $NaHCO_3$-Lösung und 5 ml Sole gewaschen, über Natriumsulfat getrocknet und am Rotationsverdampfer eingeengt. Das Rohprodukt wird an 6 g Kieselgel mit Toluol/Essigsäureethylester 9:1 bis 4:1 chromatographisch gereinigt. Man erhält die Titelverbindung vom Smp. 78-79°C (Petrolether). $[\alpha]_D^{20}$ = +108° (c = 0,5, $CHCl_3$). $R_f$-Wert 0,43 (Merck Fertigplatten), Toluol/Essigester 1:1, Ninhydrin als Entwicklungsreagens.

Die gleiche Verbindung kann auch wie folgt erhalten werden:

b) Analog dem Verfahren a) ausgehend von 131,3 mg (0,3 mmol) (3R, 4R, 1'R)-3-{1-[Dimethyl(2,3-dimethylbut-2-yl)silyloxy]ethyl}-4-(3,4-dimethoxybenzoyloxy)azetidin-2-on (>10:1 Gemisch vom Beispiel 4) nach chromatographischer Reinigung des Rohproduktes, (Kieselgel, Toluol/Ethylacetat 8:1 bis 4:1).

Beispiel 11: (5R,6S,1'R)-6-[1'-(Dimethyl-2,3-dimethylbut-2-ylsilyloxy)ethyl]-2-allyloxycarbonylaminomethyl-2-penem-3-carbonsäureallylester

Eine Lösung von 692 mg (1,6 mMol) (3S,4R,1'R)-3-[1'-(Dimethyl-2,3-dimethylbut-2-ylsilyloxy)ethyl]-4-N-allyloxycarbonylglycylthioazetidin-2-on in 9 ml Methylenchlorid wird bei -20° unter Argon nacheinander mit 360 mg (ca. 2,41 mMol) Oxalsäureallylesterchlorid und 410 ml (ca. 2,41 mMol) Hünig-Base versetzt und das entstandene Reaktionsgemisch zuerst 2,5 Stunden bei -10° und anschliessend 80 Minuten bei 0° gerührt. Es wird mit Methylenchlorid verdünnt und nacheinander mit eiskalter 0,1N HCl- und 8 %iger wässriger $NaHCO_3$-Lösung gewaschen. Der organische Anteil wird über $Na_2SO_4$ getrocknet und eingedampft. Der erhaltene Rückstand wird in 3 ml Triethylphosphit gelöst und die Lösung 2 Stunden unter Argon auf 60° erwärmt. Die flüchtigen Anteile werden zunächst unter vermindertem Druck, dann im Hochvakuum entfernt, der Rückstand in 10 ml abs. Dioxan gelöst und die resultierende Lösung unter Argon 8 Stunden auf 105° (Badtemperatur) erwärmt. Das nach Eindampfen von Dioxan unter vermindertem Druck erhaltene Rohprodukt wird auf einer Kieselgelsäule mit Hexan/Ethylacetat (9:1 und 4:1) chromatographiert. Die Titelverbindung wird als farbloses Oel erhalten. $[\alpha]$ = +62 ± 2,7° (c = 0,371 % in $CHCl_3$), U.V.: $\lambda_{max}$=320 nm ($\varepsilon$=6230; in Ethanol).

Beispiel 12: (5R,6S,1'R)-2-Aminomethyl-6-[1'-(dimethyl-2,3-dimethylbut-2-ylsilyloxy)ethyl]-2-penem-3-carbonsäure

Eine Lösung von 367 mg (0,72 mMol) (5R,6S,1'R)-6-[1'-Dimethyl-(2,3-dimethylbut-2-ylsilyloxy)ethyl]-2-allyloxycarbonylaminomethyl-2-penem-3-carbonsäureallylester in 7 ml abs. Tetrahydrofuran wird bei Raumtemperatur mit 223 mg (1,59 mMol) Dimedon und 36 mg Tetrakis-triphenylphosphin-palladium versetzt und die entstandene Lösung bei Raumtemperatur weitergerührt. Bald beginnt die Ausscheidung der kristallinen Titelverbindung und ist nach 1 Stunde praktisch beendet. Die Kristalle werden abfiltriert und auf dem Filter mit etwas Tetrahydrofuran nachgewaschen. Die erhaltene Titelverbindung bildet farblose Kristalle, F. 119°; UV: $\lambda_{max}$ (EtOH):309 nm, 268 nm.

Beispiel 13: (5R,6S,1'R)-6-(1'-Hydroxyethyl)-2-allyloxycarbonylaminomethyl-2-penem-3-carbonsäureallylester

Eine Lösung von 50 mg (5R,6S,1'R)-6-[1'-(Dimethyl-2,3-dimethylbut-2-ylsilyloxy)ethyl]-2-allyloxycarbonylaminomethyl-2-penem-3-carbonsäureallylester in 1 ml 2,6-Lutidin wird mit 60 mg (ca. 30 mMol) einer 70%igen HF-Harnstoff-Mischung versetzt und 2 Stunden bei Raumtemperatur gerührt. Dann werden zur Reaktionslösung weitere 5 Tropfen (ca. 100 mg = ca. 50 mM) HF-Harnstoff zugefügt. Das viskose Gemisch wird mit 1 ml Methylenchlorid verdünnt, über Nacht bei Raumtemperatur gerührt und erneut mit 10 Tropfen HF-Harnstoff (ca. 100 mM) versetzt. Nach weiteren 24 Stunden Reaktionszeit bei Raumtemperatur wird das Reaktionsgemisch mit Wasser verdünnt und mit Essigester extrahiert. Die organische Phase wird anschliessend mit 4N Salzsäure und mit gesättigter, wässriger Natriumchlorid-Lösung gewaschen, getrocknet, und im Rotationsverdampfer eingeengt. Durch Umlösen des anfallenden Rohproduktes aus Ether/Hexan wird die reine Titelverbindung gewonnen. F. 141-141,5°. ($R_f$ Merck Fertigplatten, Toluol/Essigester 1:1) : 0,20.

Beispiel 14: (5R,6S,1'R)-2-Aminomethyl-6-(1'-hydroxyethyl)-2-penem-3-carbonsäure

Eine Lösung von 313 mg (0,85 mmol) (5R,6S,1'R)-2-Allyloxycarbonylaminomethyl-6-(1'-hydroxyethyl)-2-penem-3-carbonsäureallylester und 174,6 mg (1,24 mmol) Dimedon in 7,5 ml Tetrahydrofuran wird während 5 Minuten mit Argon gespült und unter Argon-Atmosphäre bei Raumtemperatur mit 27 mg (0,023 mmol) Tetrakistriphenylphosphin-palladium versetzt. Nach ca. 5 Min beginnt ein Niederschlag auszu fallen, der nach weiteren 2 Stunden Rühren abfiltriert, mit ca. 10 ml Tetrahydrofuran gewaschen und anschliessend in 4 ml $H_2O$ gelöst wird. Zur beigen Lösung wird eine Mikrospatelspitze Aktivkohle und 2 Mikrospatelspitzen Tonsil gegeben, 5 Min gut gerührt und über Hyflo klar filtriert. Am Hochvakuum wird eingeengt und der erhaltene dicke Kristallbrei mit 3 ml kaltem Ethanol versetzt und abgenutscht. Die farblosen Kristalle werden mit 2 ml Tetrahydrofuran gewaschen und am Hochvakuum getrocknet. Man erhält die Titelverbindung vom Rf-Wert = 0,50 (DC:$H_2O$, OPTI $UPC_{12}$); $[\alpha]_D^{20} = +176°$ (c = 0,5, $H_2O$).

Beispiel 15: (3R)-3-(tert.-Butyl-dimethylsilyloxy)but-1-en-1-yl-pivaloat

Analog Beispiel 1 wird ausgehend von 24,4 g (121 mmol) (3R)-3-(tert.-Butyl-dimethylsilyloxy)butyraldehyd und 38,3 ml (311 mmol) Pivalinsäurechlorid die Titelverbindung (Sdp. 65-70° C/0,05 Torr) als Gemisch von E/Z Isomeren im Verhältnis von >10:1 erhalten.

IR ($CH_2Cl_2$) 2860, 1740, 1140. $^1H$-NMR (60 MHz, $CDCl_3$) der Hauptkomponente zeigt Signale u.a. bei 7,22 ppm (1H, d x d, $J_1$ = 12 Hz, $J_2$ = 1,5 Hz); 5,4 ppm (1H, d x d, $J_1$ = 12 Hz, $J_2$ = 6 Hz), 4,3 ppm (1H, 5-Linien System).

Das Ausgangsmaterial ist bekannt.

Beispiel 16: (3R,4R,1'R)-3-[1-(tert.-Butyl-dimethylsilyloxy)ethyl]-4-pivaloyloxyazetidin-2-on

Analog Beispiel 3 wird ausgehend von 14,3 g (50 mmol) (3R)-3-(tert.-Butyl-dimethylsilyloxy)but-1-en-1-yl-pivaloat (E:Z >10:1) und 10 ml (114 mmol) Chlorsulfonylisocyanat die Titelverbindung als 96:4 Gemisch von Diastereoisomeren mit (3R,4R,1'R)-respektive (3S,4S,1'R)-Konfiguration erhalten. Tieftemperaturumkristallisation aus Petrolether bei -50° C ergibt das reine (3R,4R,1'R)-Diastereomere,

$[\alpha]_D^{20} = +50°$ (c = 0,5, $CHCl_3$), Smp. 90-92° C, IR ($CH_2Cl_2$) 3410, 2960, 2930, 2860, 1790, 1732, 1140 $cm^{-1}$. Charakteristische Signale im 360 MHz-$^1H$-NMR ($CDCl_3$): 5,8 ppm (1H, d, J = 1,4 Hz), 3,2 ppm (1H, d x d, $J_1$ = 1,4 Hz, $J_2$ = 4,0 Hz), 1,26 ppm (d, J = 6,5 Hz).

Die Titelverbindung kann auch bei Normaldruck sublimiert werden.

Beispiel 17: (3R,4R,1'R)-3-(1-Hydroxyethyl)-4-pivaloyloxyazetidin-2-on

Analog Beispiel 6 wird ausgehend von 329,5 mg (1,0 mmol) (3R,4R,1'R)-3-[1-(tert.-Butyl-dimethylsilyloxy)ethyl]-4-pivaloyloxyazetidin-2-on (verunreinigt mit <10 % des (3S,4S,1'R)-Diastereomeren) die Titelverbindung erhalten. Smp. 132-133° C ($CH_2Cl_2$/Petrolether), $[\alpha]_D^{20} = +48°C$ (c = 0,5, $CHCl_3$).

**Patentansprüche**

1. Verfahren zur Herstellung von β-Lactamverbindungen der Formel

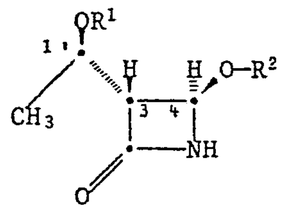

(I),

gegebenenfalls im Gemisch mit dem (3S,4S,1'R)-Diastereomeren (Ia), worin $R^1$ Wasserstoff oder eine

unter den Ringschlussbedingungen nicht abspaltbare Hydroxyschutzgruppe R1a und $R^2$ den Acylrest einer schwachen Carbonsäure darstellt, dadurch gekennzeichnet, dass man eine Verbindung der Formel

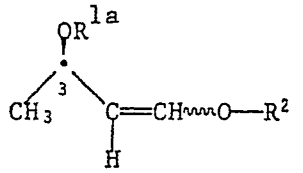

(II),

worin R1a und $R^2$ die unter Formel I angegebenen Bedeutungen haben, mit Chlorsulfonylisocyanat umsetzt, aus dem gebildeten Produkt die Chlorsulfongruppierung, reduktiv abspaltet, gewünschtenfalls die Hydroxyschutzgruppe R1a abspaltet, und gewünschtenfalls die gebildete Verbindung der Formel I von dem vorhandenen (3S,4S,1'R)-Diastereomeren befreit.

2. Verfahren nach Anspruch 1, zur Herstellung von Verbindungen der Formel I, worin $R^1$ Wasserstoff ist.

3. Verfahren nach Anspruch 1, zur Herstellung von Verbindungen der Formel I, worin $R^1$ gegebenenfalls durch Halogen in 2-Stellung substituiertes Niederalkanoyl, z.B. Acetyl, Chloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromethoxycarbonyl oder 2,2,2-Trichlorethoxycarbonyl, Niederalkenyloxycarbonyl, z.B. Allyloxycarbonyl, oder gegebenenfalls durch Nitro substituiertes Phenylniederalkoxycarbonyl, z.B. 4-Nitrobenzyloxycarbonyl, 2-Halogenniederalkyl, z.B. 2-Chlor-, 2-Brom-, 2-Jod- und 2,2,2-Trichlorethyl, oder gegebenenfalls durch Halogen, z.B. Chlor, Niederalkoxy, z.B. Methoxy, und/oder Nitro substituiertes Phenylniederalkyl, wie entsprechendes Benzyl ist.

4. Verfahren nach Anspruch 1, zur Herstellung von Verbindungen der Formel I, worin $R^1$ trisubstituiertes Silyl, wie Triniederalkylsilyl, z.B. Triisopropylsilyl, 2,3-Dimethylbut-2-yl-dimethylsilyl oder tert.-Butyl-dimethylsilyl ist.

5. Verfahren nach Anspruch 1, zur Herstellung von Verbindungen der Formel I, worin $R^2$ der Acylrest einer schwachen Carbonsäure ist.

6. Verfahren nach Anspruch 1, zur Herstellung von Verbindungen der Formel I, worin $R^2$ Triniederalkylacetyl, wie Pivaloyl, 2,2-Dimethylpropionyl, oder Triethylacetyl, oder unsubstituiertes oder durch Substituenten 1. Ordnung ein- oder mehrfach, z.B. zwei- oder dreifach substituiertes Benzoyl, wie 4-Methoxy-, 2,4-oder 3,4-Dimethoxy- oder 3,4,5- oder 2,4,6-Trimethoxybenzoyl oder 4-Dimethylaminobenzoyl ist.

7. Verfahren nach Anspruch 1, zur Herstellung von Verbindungen der Formel I, worin $R^2$ Pivaloyl oder 3,4-Dimethoxybenzoyl ist.

8. Verfahren nach Anspruch 1, dadurch gekennzeichnet, dass man die Reaktion in einem inerten Lösungsmittel zwischen etwa -70° C und Raumtemperatur durchführt und das erhaltene 1-Chlorsulfonylazetidinon mit einem Metallhydrid oder Raney Nickel reduziert.

9. Verfahren nach Anspruch I dadurch gekennzeichnet, dass man aus einer erhaltenen Verbindung worin $R^1$ eine Hydroxyschutzgruppe R1aist, diese abspaltet.

10. Verbindungen der Formel

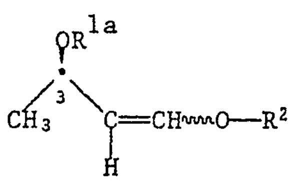

(II),

worin R1a und $R^2$ die in Anspruch 1 genannten Bedeutungen haben.

11. Verbindungen der Formel II nach Anspruch 10, worin $R^1$ gegebenenfalls durch Halogen in 2-Stellung substituiertes Niederalkanoyl, z.B. Acetyl, Chloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromethoxycarbonyl oder 2,2,2-Trichlorethoxycarbonyl, Niederalkenyloxycarbonyl, z.B. Allyloxycarbonyl, oder gegebenenfalls durch Nitro substituiertes Phenylniederalkoxycarbonyl, z.B. 4-Nitrobenzyloxycarbonyl, 2-Halogenniederalkyl, z.B. 2-Chlor-, 2-Brom-, 2-Jod- und 2,2,2-Trichlorethyl, oder gegebenenfalls durch Halogen, z.B. Chlor, Niederalkoxy, z.B. Methoxy, und/oder Nitro substituiertes Phenylniederalkyl, wie entsprechendes Benzyl ist.

12. Verbindungen der Formel II nach Anspruch 10, worin $R^1$ trisubstituiertes Silyl, wie Triniederalkylsilyl, z.B. Triisopropylsilyl, 2,3-Dimethylbut-2-yl-dimethylsilyl oder tert.-Butyl-dimethylsilyl ist.

13. Verbindungen der Formel II nach Anspruch 10, worin $R^2$ der Acylrest einer schwachen Säure ist.

14. Verbindungen der Formel II nach Anspruch 10, worin $R^2$ Triniederalkylacetyl, wie Pivaloyl,

2,2-Dimethylpropionyl, oder Triethylacetyl, oder unsubstituiertes oder durch Substituenten 1. Ordnung ein- oder mehrfach, z.B. zwei- oder dreifach substituiertes Benzoyl, wie 4-Methoxy-, 2,4- oder 3,4-Dimethoxy- oder 3,4,5- oder 2,4,6-Trimethoxybenzoyl oder 4-Dimethylaminobenzoyl oder der Acylrest eines Kohlensäurehalbesters -CO-O-R, oder einer substituierten Carbaminsäure -CO-NRR ist, worin R Niederalkyl, wie Methyl oder Ethyl, Aryl, wie Phenyl, oder Arylniederalkyl, wie Benzyl ist.

15. Verbindungen der Formel II nach Anspruch 10, worin $R^2$ Pivaloyl oder 3,4-Dimethoxybenzoyl ist.

16. Verfahren zur Herstellung von Verbindungen der Formel II nach Anspruch 11, dadurch gekennzeichnet, dass man einen Butyraldehyd der Formel

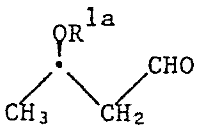

(III)

mit einer den Acylrest einer Carbonsäure der Formel

$HO-R^2$ (IV)

einführenden Mittel verestert und gewünschtenfalls das trans (E)-Isomer) aus dem erhältlichen Gemisch des trans (E)- und cis (Z)-Isomeren isoliert.

17. Verwendung einer Verbindung der Formel I, gegebenenfalls im Gemisch mit dem (3S,4S,1'R)-Diastereomeren (Ia) nach Anspruch 1, in einem Verfahren zur Herstellung von Verbindungen der Formel

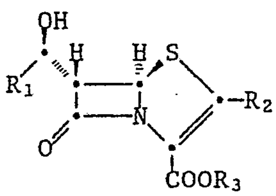

(I'),

oder Salzen davon, dadurch gekennzeichnet, dass man

a) eine Verbindung der Formel I, gegebenenfalls im Gemisch mit einer Verbindung der Formel Ia, mit einer den Thiocarbonsäurerest der Formel

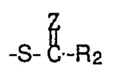

(III')

einführenden Verbindung behandelt,

b) eine erhältliche Verbindung der Formel

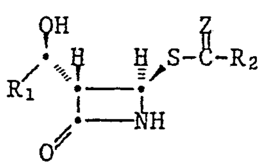

(IV')

mit einem den Acylrest eines Oxalsäurehalbesters der Formel

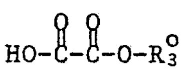

(V')

einführenden Acylierungsmittel behandelt,

c) eine erhältliche Verbindung der Formel

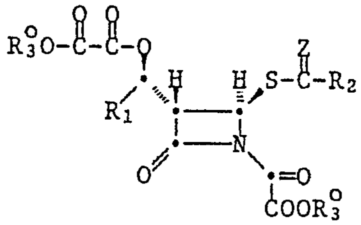

(VII')

mit einer organischen Verbindung des dreiwertigen Phosphors behandelt, und
d) in einer erhältlichen Verbindung der Formel

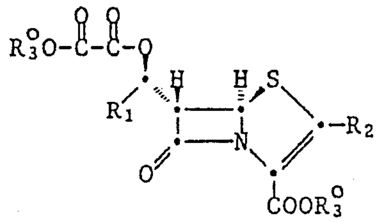

(VIII'),

die Oxalylestergruppe -CO-COO-$R_3^O$ und gewünschtenfalls die Schutzgruppe $R_3^O$ und/oder eine gegebenenfalls im Rest $R_2$ vorhandene Schutzgruppe $R_2^O$ in beliebiger Reihenfolge abspaltet und durch Wasserstoff ersetzt, und gewünschtenfalls eine erhältliche Verbindung der Formel I mit salzbildender Gruppe in ein Salz oder ein erhältliches Salz in die freie Verbindung überführt, in welchen Verbindungen $R_1$ Methyl, $R_2$ ein organischer Rest, der eine funktionelle Gruppe tragen kann, die gegebenenfalls durch eine übliche Schutzgruppe $R_2^O$ geschützt sein kann, $R_3$ Wasserstoff oder eine übliche Carboxylschutzgruppe $R_3^O$, und Z Sauerstoff oder Schwefel darstellen.

18. Verfahren zur Herstellung von Verbindungen der Formel IV', worin die l'-Hydroxygruppe gegebenenfalls durch die Gruppe $R^{1a}$ geschützt sein kann, dadurch gekennzeichnet, dass man eine Verbindung der Formel I, gegebenenfalls im Gemisch mit einer Verbindung der Formel Ia, mit einer den Thiocarbonsäurerest der Formel -SC(=Z)-$R_2$ (III') einführenden Verbindung behandelt.

19. Die neuen Verbindungen der Formel I, gegebenenfalls im Gemisch mit dem (3S,4S,1'R)-Diastereomeren der Formel Ia.

20. Verbindungen gemäss Anspruch 19, worin $R^1$ Wasserstoff oder Dimethyl(2,3-dimethylbut-2-yl)silyl ist.

21. Verbindungen gemäss Anspruch 19, worin $R^2$ Pivaloyl oder 3,4-Dimethoxybenzoyl ist.

22. (3R, 4R, 1'R)-3-{1-[Dimethyl(2,3-dimethylbut-2-yl)silyloxy]ethyl}-4-pivaloyloxyazetidin-2-on, gegebenenfalls im Gemisch $>$10:1 mit seinem (3S,4S,1'R)-Diastereomeren gemäss Anspruch 19.

23. (3R, 4R, 1'R)-3-{1-[Dimethyl(2,3-dimethylbut-2-yl)silyloxy]ethyl}-4-(3,4-dimethoxybenzoyloxy)azetidin-2-on, gegebenenfalls im Gemisch $>$10:1 mit seinem (3S,4S,1'R)-Diastereomeren gemäss Anspruch 19

24. (3R, 4R, 1'R)-3-[1-(tert.-Butyl-dimethylsilyloxy)ethyl]-4-pivaloyloxyazetidin-2-on, gegebenenfalls im Gemisch mit seinem (3S,4S,1'R)-Diastereomeren gemäss Anspruch 19.

Europäisches Patentamt

# EUROPÄISCHER RECHERCHENBERICHT

Nummer der Anmeldung

EP 87 81 0497

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 167 155 (KANEGAFUCHI K.K.K.)<br>* Insgesamt *<br>--- | 1-18 | C 07 F 7/18<br>C 07 D 205/08<br>C 07 D 499/00 |
| D,Y | EP-A-0 179 318 (HOFFMANN-LA ROCHE)<br>* Ansprüche *<br>--- | 1-18 | |
| Y | EP-A-0 181 831 (CIBA-GEIGY)<br>* Ansprüche *<br>--- | 1,19-24 | |
| Y | TETRAHEDRON, Band 39, Nr. 14, 1983, Seiten 2399-2407, Pergamon Press Ltd, GB; M. SHIOZAKI et al.: "Stereocontrolled syntheses of chiral and racemic key intermediates to thienamycin from D-allo-threonine and trans-crotonic acid"<br>* Insgesamt *<br>--- | 1,17 | |
| P,Y | EP-A-0 230 248 (KANEGAFUCHI K.K.K.)<br>* Insgesamt *<br>----- | 1-18 | RECHERCHIERTE SACHGEBIETE (Int. Cl.4)<br>C 07 F 7/00<br>C 07 D 205/00<br>C 07 D 499/00 |

Der vorliegende Recherchenbericht wurde für alle Patentansprüche erstellt

| Recherchenort | Abschlußdatum der Recherche | Prüfer |
|---|---|---|
| DEN HAAG | 10-11-1987 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument